# EUROPEAN PATENT APPLICATION

(11) **EP 4 166 503 A1**
(43) Date of publication of application: **19.04.2023**
(21) Application number: 20939668.8
(22) Date of filing: 28.08.2020
(51) Int. Cl.: C01F 17/247, C01F 17/10, A61K 33/244

(54) **METHOD FOR PREPARING LANTHANUM CARBONATE TETRAHYDRATE AND PRODUCT THEREOF**

(30) Priority: 13.06.2020 CN 202010538820
(71) Applicant: Nanjing Cavendish Bio-Engineering Technology Co., Ltd., Nanjing, Jiangsu 210033 (CN)
(72) Inventor: XU, Yongxiang, Nanjing, Jiangsu 210033 (CN)
(74) Representative: Cameron Intellectual Property Ltd
(86) International application number: PCT/CN2020/112170
(87) International publication number: WO 2021/248702

(57) **Abstract**

A method for preparing lanthanum carbonate tetrahydrate and a product thereof. The lanthanum carbonate tetrahydrate is prepared by reacting lanthanum oxide and acetic acid with potassium carbonate or potassium bicarbonate or ammonium bicarbonate to prepare lanthanum carbonate octadhydrate, and drying the lanthanum carbonate decahydrate. Compared with the lanthanum carbonate tetrahydrate in the prior art, the prepared lanthanum carbonate tetrahydrate has a characteristic spectral peak on a terahertz spectrum, and has excellent dissociation and dissolution characteristics of lanthanum ions.

## Description

### Technical Field

The application relates to, but is not limited to, the field of medicinal technology, and in particular to a preparation method of lanthanum carbonate tetrahydrate and the product thereof.

### Background

Too much phosphate in blood, i.e. hyperphosphatemia, is one of the complications of chronic renal failure, and 80% of patients with renal dialysis suffer from this disease. In addition to bone diseases such as fractures, hyperphosphatemia can also cause cardiovascular damage. It is reported that about 50% of patients died accordingly (Shen Lei, Jin Zhan, Lanthanum carbonate, a new anti-hyperphosphatemia drug, Pharmaceutical Progress, 2005, 29 (6): 286-287).

At present, the treatment of the disease is mainly diet control and the use of phosphate binders. Phosphate binders can chelate phosphorus in food and prevent phosphorus absorption in gastrointestinal tract. Commonly used phosphate binders include aluminum-containing phosphorus binders, such as aluminum hydroxide; calcium-containing phosphorus binders, such as calcium carbonate and calcium acetate; and magnesium-containing phosphorus binders, such as magnesium carbonate. Among them, aluminum hydroxide is the phosphorus-reducing drug with the strongest phosphorus binding force. However, it has been found in long-term clinical research that aluminum poisoning is prone to occur when aluminum-containing formulations are administered, and they are rarely used at present. When using calcium salts, long-term administration is prone to cause flatulence, belching and constipation, leading to cardiovascular diseases such as cardiovascular calcification. When magnesium-containing phosphorus binders are used, it is easy to cause hypermagnesemia in patients with renal failure.

In addition, Shire Company has developed and commercialized lanthanum carbonate under the trade name Fosrenol. As a non-calcium and non-aluminum phosphate binder, lanthanum carbonate has the same effect as traditional binders on reducing phosphate level in patients' serum.

Anormed Inc. disclosed in Chinese patent CN1102393C that lanthanum carbonate tetrahydrate can be administered via gastrointestinal tract to treat hyperphosphatemia in patients with renal failure; meanwhile, the invention also disclosed a preparation method of lanthanum carbonate tetrahydrate: lanthanum oxide reacts with nitric acid or hydrochloric acid to obtain lanthanum nitrate or lanthanum chloride, which then reacts with sodium carbonate and undergoes calcination to obtain lanthanum carbonate tetrahydrate. The lanthanum carbonate tetrahydrate prepared by the preparation method has the problem that sodium ion residue exceeds 250 ppm. However, the residue and intake of sodium ions will increase the kidney burden of patients with renal disease.

### Summary of the Invention

The following is an overview of the subject matter described in detail herein. This summary is not intended to limit the protection scope of the claims.

The present application provides a preparation method of lanthanum carbonate tetrahydrate, and the product obtained by the preparation method has a stable crystal form, the product is easy to handle, and the product possesses excellent dissolution performance.

The present application provides a preparation method of lanthanum carbonate tetrahydrate, which includes the following steps:
(1) reacting lanthanum oxide with acetic acid to obtain lanthanum acetate;

   La₂O₃ + CH₃COOH → La(CH₃COO)₃
(2) reacting the lanthanum acetate obtained in step (1) with potassium carbonate or potassium bicarbonate or ammonium bicarbonate to obtain lanthanum carbonate octahydrate;

   La(CH₃COO)₃ → La₂(CO₃)₃·8H₂O
(3) drying the lanthanum carbonate octahydrate obtained in step (2) to obtain lanthanum carbonate tetrahydrate;

   La₂(CO₃)₃·8H₂O → La₂(CO₃)₃·4H₂O

In the preparation method of the embodiments of the present application, the weight ratio of lanthanum oxide to acetic acid in step (1) may be 1: 1 to 1: 1.3, preferably 1: 1.1 to 1: 1.2.

In the preparation method of the embodiments of the present application, the reaction temperature in step (1) may be 40 °C to 100 °C, preferably 60 °C to 80 °C; the reaction time may be 0.1 h to 2 h, preferably 0.5 h to 1 h.

In the preparation method of the embodiments of the present application, the weight ratio of lanthanum acetate to potassium carbonate or potassium bicarbonate or ammonium bicarbonate in step (2) may be 1: 1.2 to 1: 1.8, preferably 1: 1.4 to 1: 1.5.

In the preparation method of the embodiments of the present application, the reaction temperature in step (2) may be 30 °C to 60 °C, preferably 40 °C to 50 °C; the reaction time may be 1 h ~ 4 h, preferably 2 h to 3 h.

In the preparation method of the embodiments of the present application, the temperature for the drying in step (3) may be 60 °C to 90 °C, preferably 70 °C to 80 °C.

In another aspect, the present application provides lanthanum carbonate tetrahydrate obtained by the aforementioned preparation method.

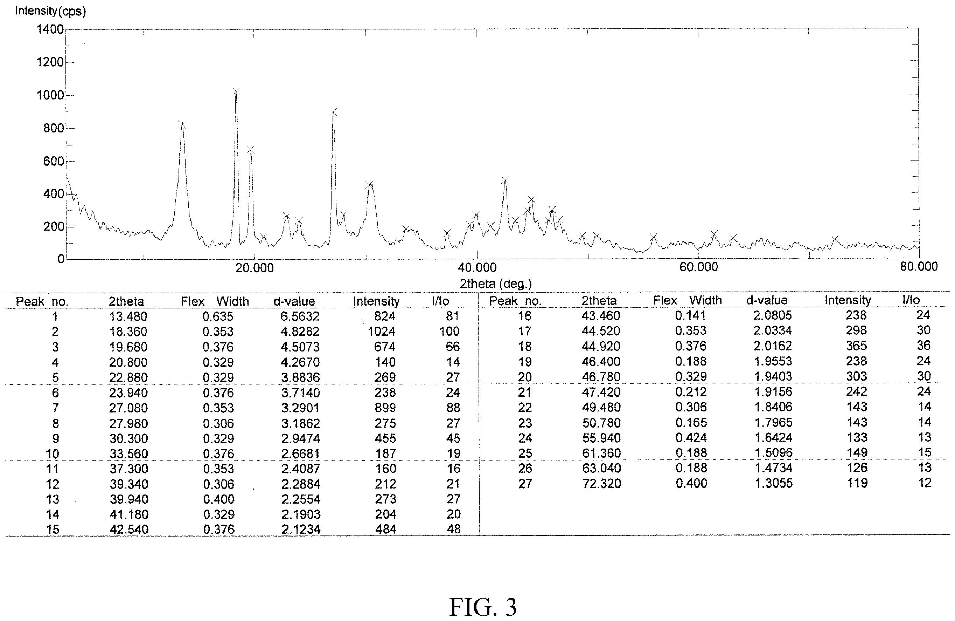

In an embodiment of the present application, the X-ray powder diffraction (X-RPD) pattern of the lanthanum carbonate tetrahydrate active pharmaceutical ingredient (API) provided by the present application is substantially as shown in Fig. 3.

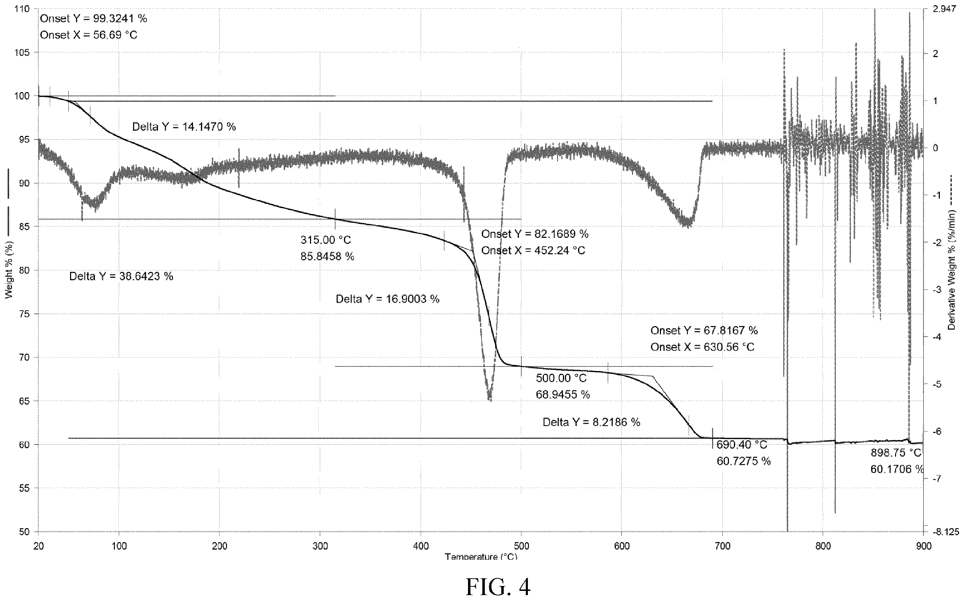

In an embodiment of the present application, the thermogravimetric analysis graph of the lanthanum carbonate tetrahydrate API provided by the present application is substantially as shown in Fig. 4.

In an embodiment of the present application, in the terahertz spectrum, the lanthanum carbonate tetrahydrate provided by the present application has no characteristic spectral peak between 1.0 THz and 3.0 THz, and has characteristic spectral peaks between 3.0 THz and 3.9 THz.

In an embodiment of the present application, the lanthanum carbonate tetrahydrate obtained by the preparation method has a sodium content of less than 0.005 wt%.

Additional features and advantages of the present application will be set forth in the description which follows, and in part will become more apparent from the description, or may be learned by practice of the application. Other advantages of the present application can be realized and obtained by embodiments described in the description.

### Brief Description of Drawings

The accompanying drawings are used to provide an understanding of the technical schemes of the present application, and constitute a part of the specification. The drawings are used to explain the technical schemes of the present application together with the embodiments of the present application, and do not constitute a limitation to the technical schemes of the present application.

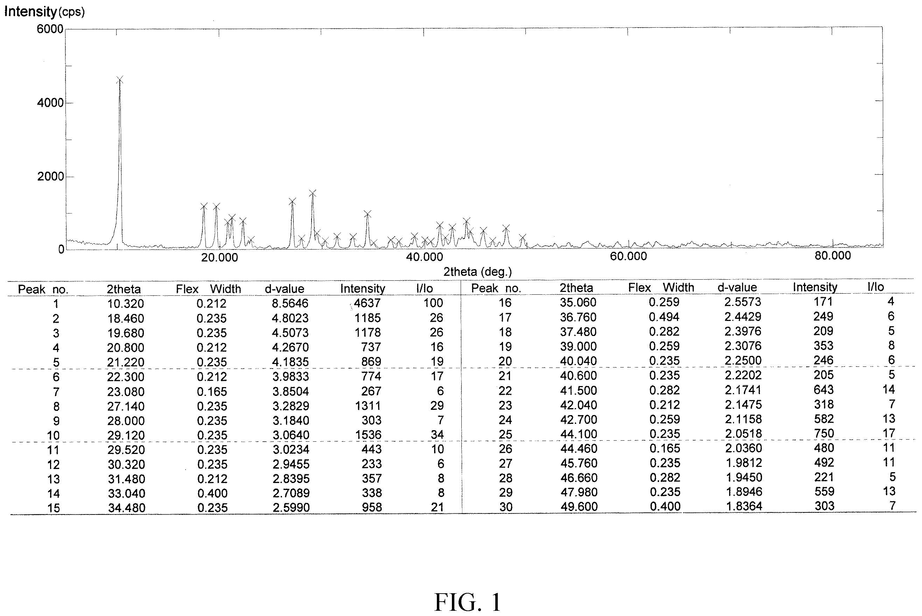

Fig. 1 shows a representative X-ray powder diffraction (XRPD) pattern of lanthanum carbonate octahydrate of Example 1 of the present application;

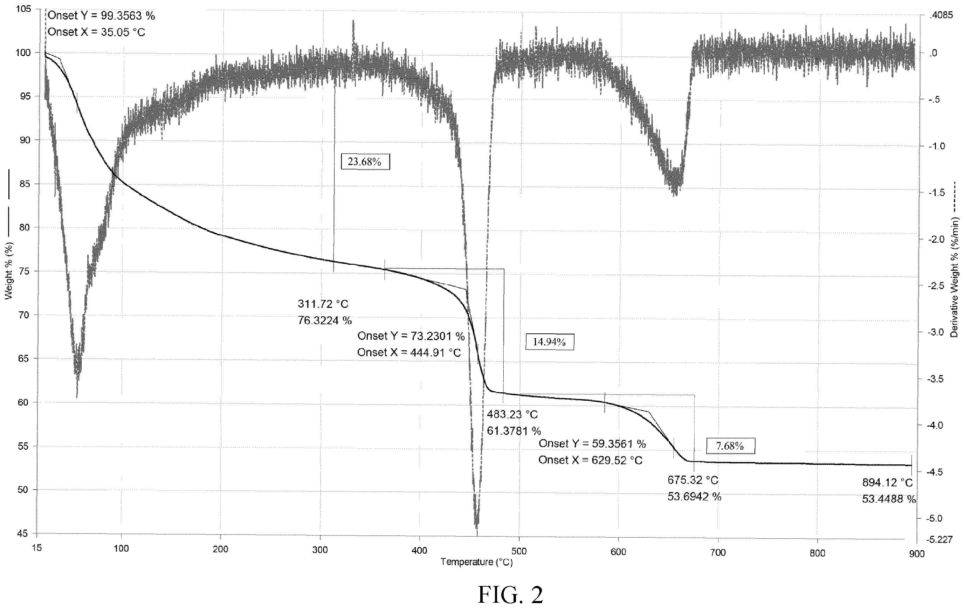

Fig. 2 shows a representative thermogravimetric analysis (TGA) graph of lanthanum carbonate octahydrate of Example 1 of the present application;
Fig. 3 shows a representative X-ray powder diffraction (XRPD) pattern of lanthanum carbonate tetrahydrate of Example 1 of the present application;
Fig. 4 shows a representative thermogravimetric analysis (TGA) graph of lanthanum carbonate tetrahydrate of Example 1 of the present application;
Fig. 5 shows representative terahertz test spectrum of lanthanum carbonate tetrahydrate of Examples 1 to 3 of the present application;
Fig. 6 shows representative terahertz test spectrum of lanthanum carbonate tetrahydrate of Comparative Examples 1 to 3;
Fig. 7 shows a representative X-ray powder diffraction (XRPD) pattern of lanthanum carbonate octahydrate with abnormal crystal form of Comparative Examples 5 to 10 of the present application;
Fig. 8 shows a representative X-ray powder diffraction (XRPD) pattern of lanthanum carbonate tetrahydrate with abnormal crystal form of Comparative Examples 5 to 10 of the present application.

### Detailed Description

To further elaborate the purpose, technical solutions and advantages of the present application, examples of the present application will be described in detail below. It should be noted that the following examples of the present application and the features of the examples may be arbitrarily combined with each other provided that there is no conflict.

In the embodiments of the present application, the terahertz testing instrument is: Brooke V80V Fourier infrared spectrometer, 25 µm beam splitter, spectral testing range (0.5 THz to 4 THz), and testing accuracy of 2 cm⁻¹.

### Test environment: vacuum

Test method: Solid tableting: mixing pure sample with polyethylene powder in a certain ratio, grinding evenly, pressing into thin tablet and testing (the weight ratio of the sample to polyethylene is 1: 3, with 12 mg in total, the diameter of the thin tablet is 13 mm and the thickness is about 1 mm).

The water used in the examples is double redistilled water, and the residual sodium ions are all less than 10 ppm.

XRPD is measured using a rotating target X-ray diffractometer, manufacturer: Japan Rigaku Corporation, model: D/max 2500/PC. Test conditions: tube pressure: 40 kV, tube current: 100 mA, Cu target, graphite curved monochromator, continuous scanning, step length of scanning: 0.02°, scanning range: 3° to 40° (2 0), slit system: DS = SS = 1°, RS = 0.3 mm.

TGA is measured using PYRIS 1 TGA manufactured by PE Perkin Elmer.

The content of sodium ions is measured by ICP-MS in the fourth part of Chinese Pharmacopoeia, 2015 version:
Instrument: Agilent 7900 ICP-MS (with nickel sampler cone, MicroMist quartz atomizer)
ELGA PureLab Ultrapure Water Instrument
AB135-S Type Hundred-thousandth Electronic Balance
Reagent: CNW^{®} Nitric acid for trace analysis, 67% to 70%;
Ultrapure water, self-made;
Plasma gas, the carrier gas is argon, and collision gas is helium, all of which are gases of high purity (> 99.999%).
Acetic acid in the present application generally refers to pure acetic acid with a mass concentration of more than 99.5%.

### Example 1

### Preparation of Lanthanum Acetate Solution:

Lanthanum oxide (150 g) was suspended in double distilled deionized water (2826 ml), heated to 70 °C, and an aqueous solution of acetic acid (prepared from 174 g of acetic acid and 174 ml of double distilled deionized water) was added dropwise under stirring. After completion of the dropwise addition, the reaction was stirred for 0.5 h, cooled down, and filtrated under vacuum, and the filtrate was collected to obtain lanthanum acetate solution.

### Preparation of lanthanum carbonate octahydrate:

The prepared lanthanum acetate solution was heated to 50 °C and a potassium carbonate solution (prepared from 216 g of potassium carbonate and 300 ml of double distilled deionized water) was added dropwise under stirring. After completion of the dropwise addition, the reaction was kept warm and stirred for 3 h. The temperature was lowered, the reaction mixture was filtrated under vacuum, and the filter cake was rinsed with deionized water (900 ml). The resulting solid was added to double distilled deionized water (3000 ml), the temperature was controlled to 30 °C, stirred for 1 h, the temperature was lowered, and the mixture was filtrated under vacuum, double distilled deionized water (3000 ml) was added into the filter cake, and the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and filtrated under vacuum, the filter cake was rinsed with double distilled deionized water (900 ml), and dried in a blast oven at 40 °C to obtain the target compound lanthanum carbonate octahydrate 255.78 g with a yield of 92.29%.

### Preparation of lanthanum carbonate tetrahydrate:

The prepared lanthanum carbonate octahydrate (240 g) was dried in a blast oven at 80 °C, and was weighed every 2 hours to monitor the weight reduced. The target compound of 207.24 g with a yield of 98.09% was obtained.

The prepared lanthanum carbonate tetrahydrate API was treated until the D90 of the particle size is less than or equal to 30 µm, and the chewable tablets were prepared according to the following formulation:

| Name of raw materials and auxiliary materials | Amount of the formulation (g) |
|---|---|
| Lanthanum carbonate tetrahydrate | 954 (500 g in lanthanum) |
| Starch hydrolyzed oligosaccharide | 1068 |
| Silica | 42 |
| Magnesium stearate | 20 |

### Preparation process:

Lanthanum carbonate tetrahydrate, starch hydrolyzed oligosaccharide, and silica were uniformly mixed, pressed and molded by extrusion equipment, crushed, and magnesium stearate was added, mixed for about 5 minutes, and compressed into tablets with target weight.

### Example 2

### Preparation of Lanthanum Acetate Solution:

Lanthanum oxide (150 g) was suspended in double distilled deionized water (2826 ml), heated to 70 °C, and an aqueous solution of acetic acid (prepared from 174 g of acetic acid and 174 ml of double distilled deionized water) was added dropwise under stirring. After completion of the dropwise addition, the reaction was stirred for 0.5 h, cooled down, and filtrated under vacuum, and the filtrate was collected to obtain lanthanum acetate solution.

### Preparation of lanthanum carbonate octahydrate:

The prepared lanthanum acetate solution was heated to 50 °C and an ammonium bicarbonate solution (prepared from 247.5 g of ammonium bicarbonate and 1000 ml of double distilled deionized water) was added dropwise under stirring. After completion of the dropwise addition, the reaction was kept warm and stirred for 3 h. The temperature was lowered, the mixture was filtrated under vacuum, and the filter cake was rinsed with double distilled deionized water (900 ml). The resulting solid was added to double distilled deionized water (3000 ml), the temperature was controlled to 30 °C, stirred for 1 h, the temperature was lowered, and the reaction mixture was filtrated under vacuum, double distilled deionized water (3000 ml) was added into the filter cake, and the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and the mixture was filtrated under vacuum, the filter cake was rinsed with double distilled deionized water (900 ml), and dried in a blast oven at 40 °C to obtain the target compound lanthanum carbonate octahydrate 254.22 g with a yield of 91.73%.

### Preparation of lanthanum carbonate tetrahydrate:

The prepared lanthanum carbonate octahydrate (240 g) was dried in a blast oven at 80 °C, and was weighed every 2 hours to monitor the weight reduced. The target compound of 205.69 g with a yield of 97.36% was obtained.

Chewable tablets were prepared according to the formulation and preparation method of the chewable tablets of Example 1.

### Example 3

### Preparation of Lanthanum Acetate Solution:

Lanthanum oxide (150 g) was suspended in double distilled deionized water (3000 ml), heated to 70 °C, and an aqueous solution of acetic acid (prepared from 174 g of acetic acid and 180 ml of double distilled deionized water) was added dropwise under stirring. After completion of the dropwise addition, the reaction was stirred for 0.5 h, cooled down, and filtrated under vacuum, and the filtrate was collected to obtain lanthanum acetate solution.

### Preparation of lanthanum carbonate octahydrate:

The lanthanum acetate solution was heated to 50 °C and a potassium bicarbonate solution (prepared from 313.8 g of potassium bicarbonate and 1000 ml of double distilled deionized water) was added dropwise under stirring. After completion of the dropwise addition, the reaction was kept warm and stirred for 3 h. The temperature was lowered, the mixture was filtrated, and the filter cake was rinsed with double distilled deionized water (900 ml). The resulting solid was added to double distilled deionized water (3000 ml), the temperature was controlled to 30 °C, stirred for 1 h, the temperature was lowered, and the reaction mixture was filtrated under vacuum, double distilled deionized water (3000 ml) was added into the filter cake, the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and the mixture was filtrated under vacuum, the filter cake was rinsed with double distilled deionized water (900 ml), and dried in a blast oven at 40 °C to obtain the target compound lanthanum carbonate octahydrate 253.20 g with a yield of 91.36%.

### Preparation of lanthanum carbonate tetrahydrate:

The prepared lanthanum carbonate octahydrate (240 g) was dried in a blast oven at 80 °C, and was weighed every 2 hours to monitor the weight reduced. The target compound of 204.11 g with a yield of 96.61% was obtained.

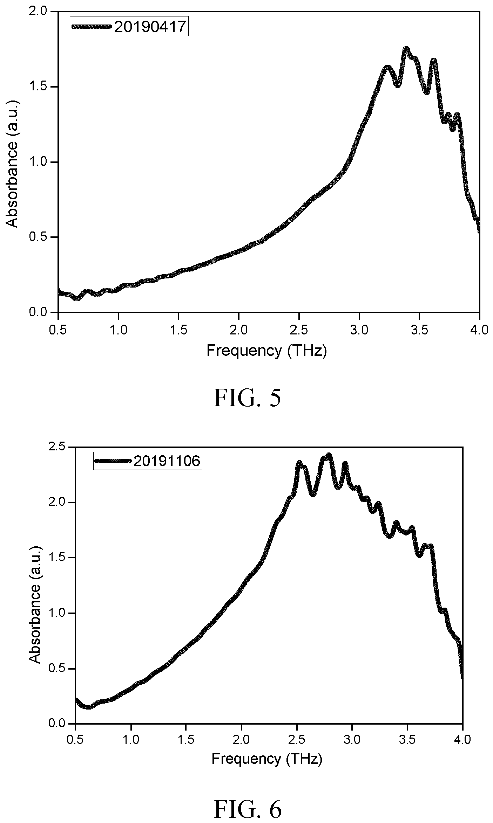

The terahertz test spectra of the lanthanum carbonate tetrahydrate prepared in Examples 1 to 3 are substantially as shown in Fig. 5.

### Comparative example 1

### Preparation of Lanthanum Acetate Solution:

Lanthanum oxide (150 g) was suspended in double distilled deionized water (2826 ml), heated to 70 °C, and an aqueous solution of acetic acid (prepared from 174 g of acetic acid and 174 ml of double distilled deionized water) was added dropwise under stirring. After completion of the dropwise addition, the reaction was stirred for 0.5 h, cooled down, and filtrated under vacuum, and the filtrate was collected to obtain lanthanum acetate solution.

### Preparation of lanthanum carbonate octahydrate:

The prepared lanthanum acetate solution was heated to 50 °C and a sodium carbonate solution (prepared from 166 g of sodium carbonate and 900 ml of double distilled deionized water) was added dropwise under stirring. After completion of the dropwise addition, the reaction was kept warm and stirred for 3 h. The temperature was lowered, the mixture was filtrated under vacuum, and the filter cake was rinsed with double distilled deionized water (900 ml). The resulting solid was added to double distilled deionized water (3000 ml), the temperature was controlled to 30 °C, stirred for 1 h, the temperature was lowered, and the reaction mixture was filtrated under vacuum, double distilled deionized water (3000 ml) was added into the filter cake, the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and the mixture was filtrated under vacuum, the filter cake was rinsed with double distilled deionized water (900 ml), and dried in a blast oven at 40 °C to obtain the target compound lanthanum carbonate octahydrate 248.25 g with a yield of 89.58%.

### Preparation of lanthanum carbonate tetrahydrate:

The lanthanum carbonate octahydrate (230 g) was dried in a blast oven at 80 °C, and was weighed every 2 hours to monitor the weight reduced. The target compound of 198.54 g with a yield of 98.06% was obtained.

Chewable tablets were prepared according to the formulation and preparation method of the chewable tablets of Example 1.

### Comparative example 2

### Preparation of Lanthanum Nitrate Solution:

Lanthanum oxide (150 g) was suspended in double distilled deionized water (2400 ml), heated to 70 ± 5 °C, and nitric acid (65% to 68% by mass, 195 ml) was added dropwise under stirring. After completion of the dropwise addition, the solution was clear. The solution was cooled down and filtrated under vacuum, and the filtrate was collected to obtain lanthanum nitrate solution.

### Preparation of lanthanum carbonate octahydrate:

The prepared lanthanum nitrate solution was heated to 40 °C and a sodium carbonate solution (prepared from 165.91 g of sodium carbonate and 900 ml of double distilled deionized water) was added dropwise under stirring. After completion of the dropwise addition, the reaction was kept warm and stirred for 1 h. The temperature was lowered, the mixture was filtrated under vacuum to obtain a filter cake. Double distilled deionized water (3750 ml) was added to the resulting solid. The temperature was controlled to 30 °C, stirred for 1 h, the temperature was lowered, and the mixture was filtrated under vacuum. This procedure was repeated six times. Then the solid was dried in a blast oven at 40 °C, obtaining the target compound lanthanum carbonate octahydrate 266.8 g with a yield of 96.27%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 209.52 g with a yield of 95.21% was obtained. XRPD shows abnormal crystal form of the product.

Chewable tablets were prepared according to the formulation and preparation method of the chewable tablets of Example 1.

### Comparative example 3

### Preparation of Lanthanum Chloride Solution:

Lanthanum oxide (150 g) was suspended in double distilled deionized water (2400 ml), heated to 70 ± 5 °C, and hydrochloric acid (36% to 38% by mass, 241.7 ml) was added dropwise under stirring. After completion of the dropwise addition, the solution was clear. The solution was cooled down and filtrated under vacuum, and the filtrate was collected to obtain lanthanum chloride solution.

### Preparation of lanthanum carbonate octahydrate:

The prepared lanthanum chloride solution was heated to 50 °C and an ammonium bicarbonate solution (prepared from 247.5 g of ammonium bicarbonate and 1000 ml of double distilled deionized water) was added dropwise under stirring. After completion of the dropwise addition, the reaction was kept warm and stirred for 3 h. The temperature was lowered, the mixture was filtrated under vacuum, and the filter cake was rinsed with double distilled deionized water (900 ml). The resulting solid was added to double distilled deionized water (3000 ml), the temperature was controlled to 30 °C, stirred for 1 h, the temperature was lowered, and the reaction mixture was filtrated under vacuum, double distilled deionized water (3000 ml) was added into the filter cake, the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and the mixture was filtrated under vacuum, the filter cake was rinsed with double distilled deionized water (900 ml), and dried in a blast oven at 40 °C to obtain the target compound lanthanum carbonate octahydrate 265.3 g with a yield of 95.73%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 215.89 g with a yield of 98.10% was obtained.

Chewable tablets were prepared according to the formulation and preparation method of the chewable tablets of Example 1.

The terahertz test spectra of the lanthanum carbonate tetrahydrate prepared in Comparative Examples 1 to 3 are substantially as shown in Fig. 6.

### Comparative example 4

### Preparation of Lanthanum Acetate Solution:

Lanthanum oxide (150 g) was suspended in double distilled deionized water (2826 ml), heated to 70 °C, and an aqueous solution of acetic acid (prepared from 174 g of acetic acid and 174 ml of double distilled deionized water) was added dropwise under stirring. After completion of the dropwise addition, the reaction was stirred for 0.5 h, cooled down, and filtrated under vacuum, and the filtrate was collected to obtain lanthanum acetate solution.

### Preparation of lanthanum carbonate octahydrate:

The prepared lanthanum acetate solution was heated to 50 °C and a sodium bicarbonate solution (prepared from 263 g of sodium bicarbonate and 2500 ml of double distilled deionized water) was added dropwise under stirring. After completion of the dropwise addition, the reaction was kept warm and stirred for 3 h. The temperature was lowered, the mixture was filtrated under vacuum, and the filter cake was rinsed with double distilled deionized water (900 ml). The resulting solid was added to double distilled deionized water (3000 ml), the temperature was controlled to 30 °C, stirred for 1 h, the temperature was lowered, and the reaction mixture was filtrated under vacuum, double distilled deionized water (3000 ml) was added into the filter cake, the temperature was controlled to 30 °C, stirred for 1 h, the temperature was lowered, and the mixture was filtrated under vacuum, double distilled deionized water (3000 ml) was added into the filter cake, the temperature was controlled to 30 °C, stirred for 1 h, the temperature was lowered, and the mixture was filtrated under vacuum, the filter cake was rinsed with double distilled deionized water (900 ml), and dried in a blast oven at 40 °C to obtain the target compound lanthanum carbonate octahydrate 244.33 g with a yield of 88.16%.

### Preparation of lanthanum carbonate tetrahydrate:

The prepared lanthanum carbonate octahydrate (230 g) was dried in a blast oven at 80 °C, and was weighed every 2 hours to monitor the weight reduced. The target compound of 200.12 g with a yield of 98.84% was obtained.

### Comparative example 5

### Preparation of Lanthanum Chloride Solution:

Lanthanum oxide (150 g) was suspended in double distilled deionized water (2400 ml), heated to 70 ± 5 °C, and hydrochloric acid (36% to 38% by mass, 241.7 ml) was added dropwise under stirring. After completion of the dropwise addition, the solution was clear. The solution was cooled down and filtrated under vacuum, and the filtrate was collected to obtain lanthanum chloride solution.

### Preparation of lanthanum carbonate octahydrate:

The prepared lanthanum chloride solution was heated to 40 °C and a sodium carbonate solution (prepared from 165.91 g of sodium carbonate and 900 ml of double distilled deionized water) was added dropwise under stirring. After completion of the dropwise addition, the reaction was kept warm and stirred for 1 h. The temperature was lowered, the mixture was filtrated under vacuum to obtain a filter cake. Double distilled deionized water (3750 ml) was added to the resulting solid. The temperature was controlled to 30 °C, stirred for 1 h, the temperature was lowered, and the mixture was filtrated under vacuum. This procedure was repeated six times. Then the solid was dried in a blast oven at 40 °C, obtaining the target compound lanthanum carbonate octahydrate 265.5 g with a yield of 95.80%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 212.45 g with a yield of 96.54% was obtained. XRPD shows abnormal crystal form of the product.

### Comparative example 6

### Preparation of Lanthanum Nitrate Solution:

Lanthanum oxide (150 g) was suspended in double distilled deionized water (2400 ml), heated to 70 ± 5 °C, and nitric acid (65% to 68% by mass, 195 ml) was added dropwise under stirring. After completion of the dropwise addition, the solution was clear. The solution was cooled down and filtrated under vacuum, and the filtrate was collected to obtain lanthanum nitrate solution.

### Preparation of lanthanum carbonate octahydrate:

The prepared lanthanum nitrate solution was heated to 40 °C and a potassium carbonate solution (prepared from 216 g of potassium carbonate and 300 ml of double distilled deionized water) was added dropwise under stirring. After completion of the dropwise addition, the reaction was kept warm and stirred for 1 h. The temperature was lowered, the mixture was filtrated under vacuum to obtain a filter cake. Double distilled deionized water (3750 ml) was added to the resulting solid. The temperature was controlled to 30 °C, stirred for 1 h, the temperature was lowered, and the mixture was filtrated under vacuum. This procedure was repeated 3 times. Then the solid was dried in a blast oven at 40 °C, obtaining the target compound lanthanum carbonate octahydrate 269.4 g with a yield of 97.21%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 215.95 g with a yield of 98.13% was obtained. XRPD shows abnormal crystal form of the product.

### Comparative example 7

### Preparation of Lanthanum Chloride Solution:

Lanthanum oxide (150 g) was suspended in double distilled deionized water (2400 ml), heated to 70 ± 5 °C, and hydrochloric acid (36% to 38% by mass, 241.7 ml) was added dropwise under stirring. After completion of the dropwise addition, the solution was clear. The solution was cooled down and filtrated under vacuum, and the filtrate was collected to obtain lanthanum chloride solution.

### Preparation of lanthanum carbonate octahydrate:

The prepared lanthanum chloride solution was heated to 40 °C and a potassium carbonate solution (216 g of potassium carbonate, 300 ml of double distilled deionized water) was added dropwise under stirring. After completion of the dropwise addition, the reaction was kept warm and stirred for 1 h. The temperature was lowered, the mixture was filtrated under vacuum to obtain a filter cake. Double distilled deionized water (3750 ml) was added to the resulting solid.

The temperature was controlled to 30 °C, stirred for 1 h, the temperature was lowered, and the mixture was filtrated under vacuum. This procedure was repeated 3 times. Then the solid was dried in a blast oven at 40 °C, obtaining the target compound lanthanum carbonate octahydrate 271.19 g with a yield of 97.85%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 217.71 g with a yield of 98.93% was obtained. XRPD shows abnormal crystal form of the product.

### Comparative example 8

### Preparation of Lanthanum Chloride Solution:

Lanthanum oxide (150 g) was suspended in double distilled deionized water (2400 ml), heated to 60 °C, and hydrochloric acid (36% to 38% by mass, 241.7 ml) was added dropwise under stirring. The pH was controlled at 3 to 5. After completion of the dropwise addition, the solution was clear. The solution was cooled down and filtrated under vacuum, and the filtrate was collected to obtain lanthanum chloride solution.

### Preparation of lanthanum carbonate octahydrate:

The prepared lanthanum chloride solution was heated to below 40 °C, for example, 30-40 °C, and a sodium bicarbonate solution (prepared from 263 g of sodium bicarbonate and 2500 ml of double distilled deionized water) was added dropwise under stirring. The pH was controlled at 6 to 7. After completion of the dropwise addition, the temperature was kept at 40-50 °C and stirred for 0.5 h. The mixture was filtrated under vacuum to obtain a filter cake. Double distilled deionized water (3750 ml) was added to the resulting solid. The temperature was controlled to 30 °C, stirred for 1 h, cooled down, and filtrated under vacuum. This procedure was repeated 3 times. Then the solid was dried in a blast oven at 40 °C, obtaining the target compound lanthanum carbonate octahydrate 254.22 g with a yield of 91.73%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 209.52 g with a yield of 95.21% was obtained. XRPD shows abnormal crystal form of the product.

### Comparative example 9

### Preparation of Lanthanum Nitrate Solution:

Lanthanum oxide (150 g) was suspended in double distilled deionized water (2400 ml), heated to 70 ± 5 °C, and nitric acid (65% to 68% by mass, 195 ml) was added dropwise under stirring. After completion of the dropwise addition, the solution was clear, and then cooled down and filtrated under vacuum, and the filtrate was collected to obtain lanthanum nitrate solution.

### Preparation of lanthanum carbonate octahydrate:

The prepared lanthanum nitrate solution was heated to below 40 °C, for example, 30-40 °C and a sodium bicarbonate solution (prepared from 263 g of sodium bicarbonate and 2500 ml of double distilled deionized water) was added dropwise under stirring. The pH was controlled at 6 to 7. After completion of the dropwise addition, the temperature was kept at 40 to 50 °C and stirred for 0.5 h. The mixture was filtrated under vacuum to obtain a filter cake. Double distilled deionized water (3750 ml) was added to the resulting solid. The temperature was controlled to 30 °C, stirred for 1 h, cooled down, and filtrated under vacuum. This procedure was repeated 3 times. Then the solid was dried in a blast oven at 40 °C, obtaining the target compound lanthanum carbonate octahydrate 267.6 g with a yield of 96.56%. XRPD shows abnormal crystal form of the product.

### Preparation of lanthanum carbonate tetrahydrate:

The prepared lanthanum carbonate octahydrate (250 g) was dried in a vacuum drying oven at 70 °C, and was weighed every 2 hours to monitor the weight reduced. The target compound of 214.33 g with a yield of 97.39% was obtained. XRPD shows abnormal crystal form of the product.

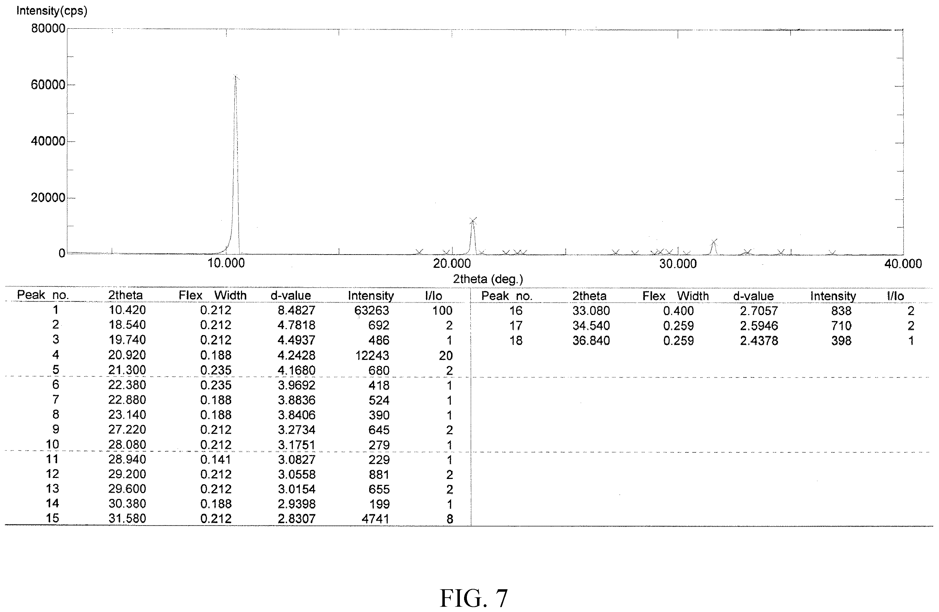

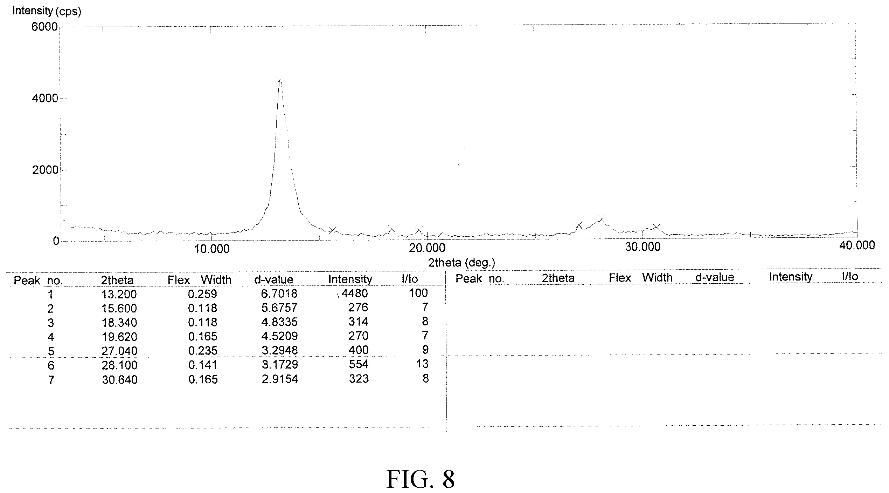

The X-ray powder diffraction (XRPD) patterns of lanthanum carbonate octahydrate with abnormal crystal form prepared in Comparative Examples 5 to 10 are substantially as shown in Fig. 7, and the X-ray powder diffraction (XRPD) patterns of lanthanum carbonate tetrahydrate with abnormal crystal form are substantially as shown in Fig. 8.

### Sodium ion content

Sodium ion detection results of lanthanum carbonate tetrahydrate API prepared in Examples 1, 2 and Comparative Examples 1, 2 and 5:

| Examples and product batch numbers | Preparation conditions | Sodium ion content in API |
|---|---|---|
| EXAMPLE 1 20190426-1 | Acetic acid, potassium carbonate | 0.6359 ppm |
| EXAMPLE 2 20190412-1 | Acetic acid, ammonium bicarbonate | 0.5490 ppm |
| Comparative Example 1 20191217-2 | Acetic acid, sodium carbonate | 578.5462 ppm |
| Comparative Example 2 20190412-2 | Nitric acid, sodium carbonate | 274.6957 ppm |
| Comparative Example 5 20190430-1 | Hydrochloric acid, sodium carbonate | 432.3226 ppm |

### Dissolution comparison

### Dissolution comparison of the tablets of Examples 1, 2 and Comparative Examples 1, 2, 3 (pH 1.0, pH 3.0, pH 4.5, pH 6.8).

Dissolution determination method: general rules 0931 the second method (paddle method) of Chinese Pharmacopoeia, 2015 version, volume IV
Rotating speed: 50 rpm
Dissolution media: hydrochloric acid medium pH 1.0, acetate buffer pH 3.0 (acetic acid-sodium acetate buffer), acetate buffer pH 4.5 (acetic acid-sodium acetate buffer), borate buffer pH 6.8 (boric acid-sodium hydroxide buffer)
Volume of dissolution medium: 900 ml
Time points of sampling: 20 min, 40 min, 60 min, 120 min, 180 min, 240 min, 360 min, 720 min, 1440 min
Volume of sampling: 7 ml (filtration with filter membrane, discarding initial filtrate, and take subsequent filtrate as the test solution)
Determination method: EDTA titration

### Results of dissolution determination:

### Comparison of the results of dissolution determination in hydrochloric acid medium pH 1.0

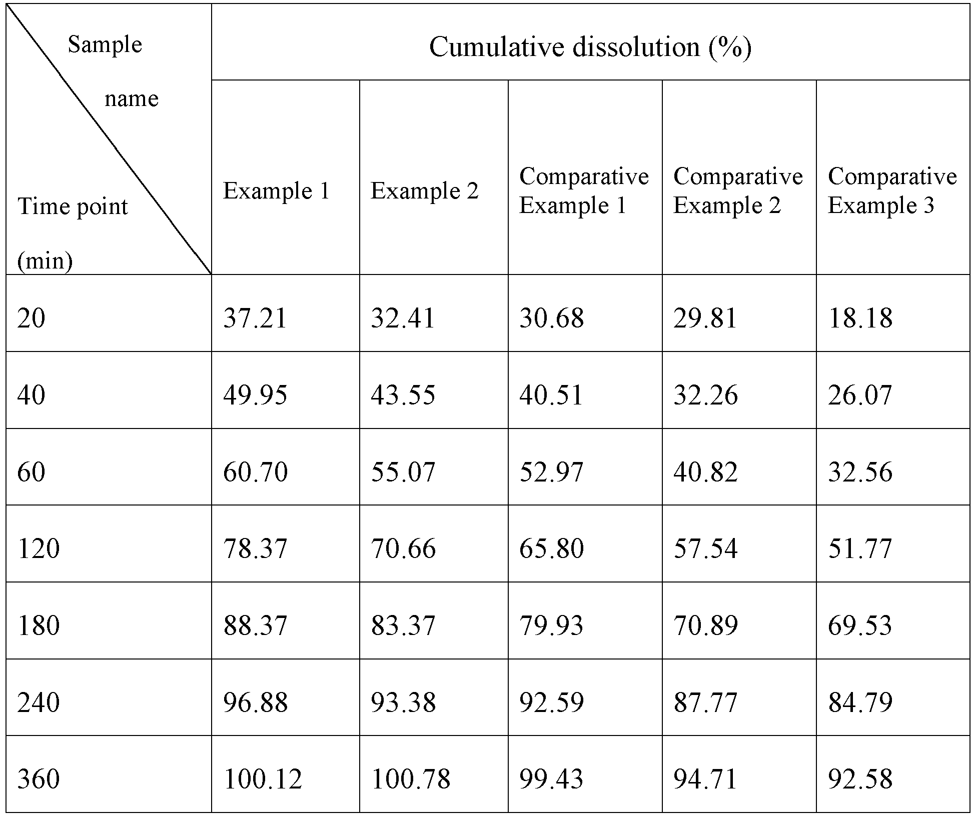

### Comparison of the results of dissolution determination in acetate buffer medium pH 3.0

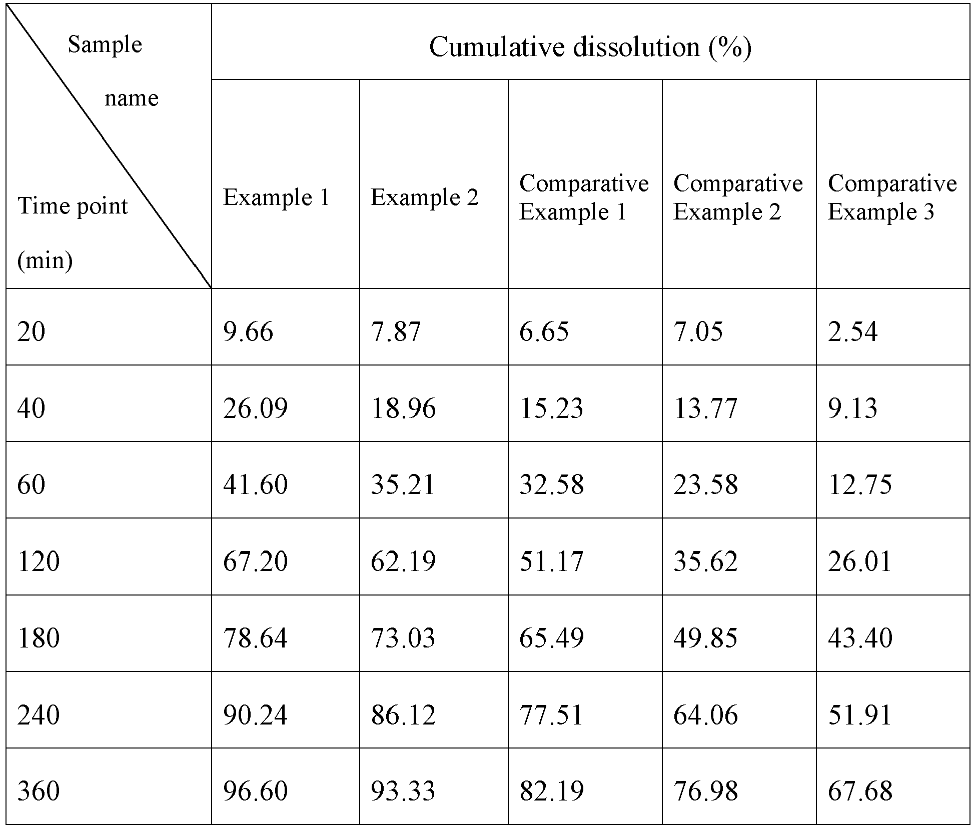

### Comparison of the results of dissolution determination in acetate buffer medium pH 4.5

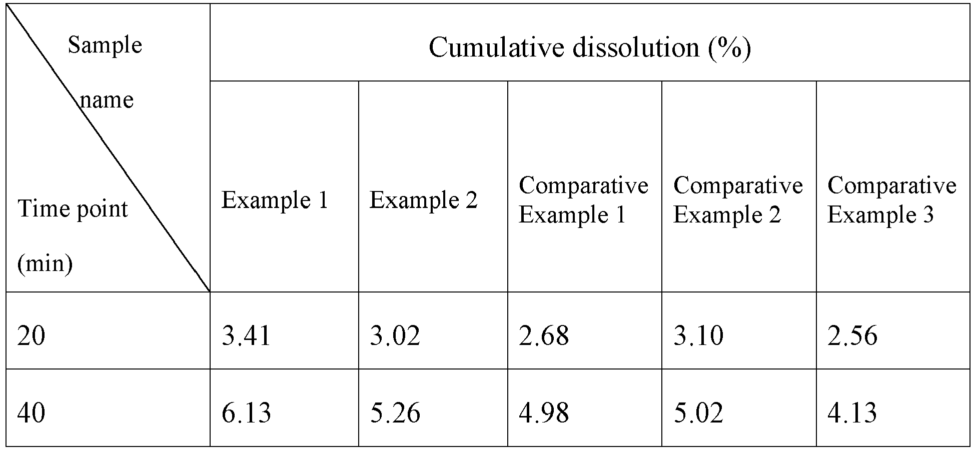

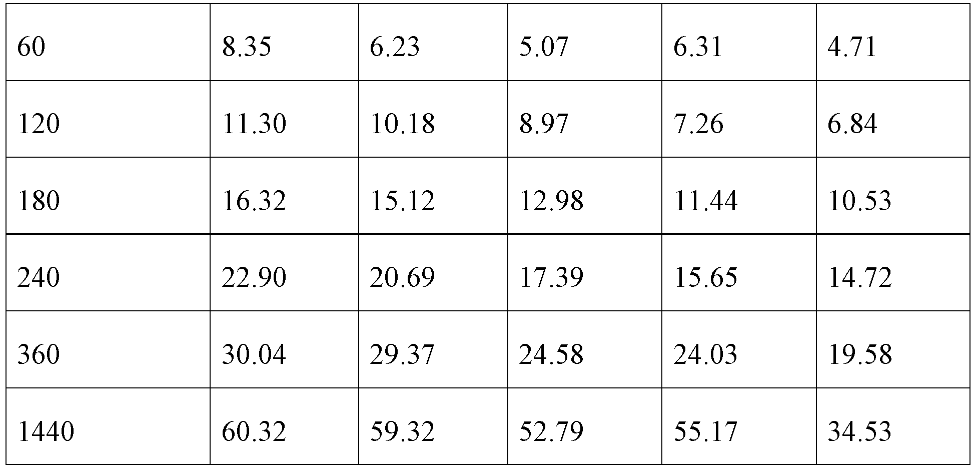

### Comparison of the results of dissolution determination in borate buffer medium pH 6.8

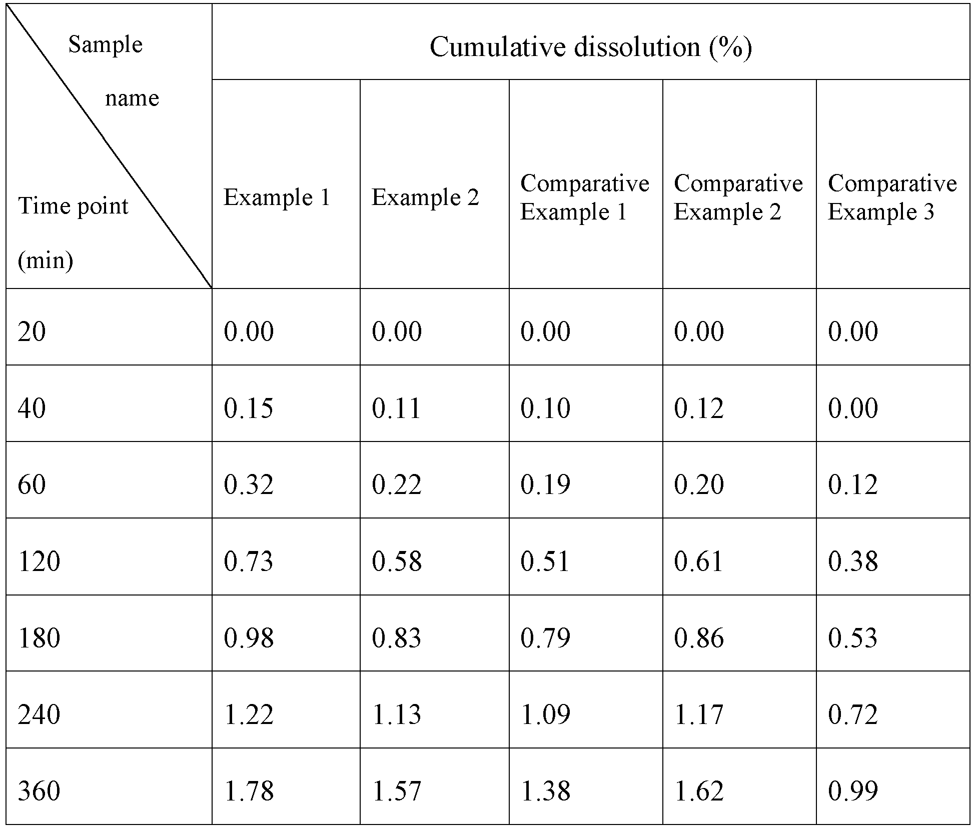

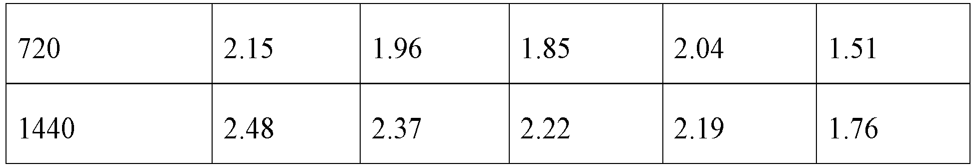

According to the dissolution study, the lanthanum carbonate tetrahydrate API prepared in the Examples of the present application has excellent dissolution characteristics. Especially under the conditions of hydrochloric acid medium pH 1.0 and acetate buffer medium pH 3.0, compared with the lanthanum carbonate tetrahydrate API prepared by the existing process, it has remarkably excellent dissolution characteristics, which can ensure the rapid dissociation of lanthanum ions after entering the gastrointestinal tract, providing sufficient ligand, lanthanum ions, for binding phosphate ions in the intestinal tract.

The disclosure is examples of the principles of the embodiments of the present application, and does not limit the present application formally or substantially in any manner, or limit the present application to specific embodiments. It is obvious to those skilled in the art that the elements, methods and systems of the technical solutions of the embodiments of the present application can be altered, changed, modified and evolved without departing from the principles, spirits and scopes of the embodiments and technical solutions of the present application as defined in the claims. These altered, changed, modified and evolved embodiments are all included in equivalent embodiments of the present application, and these equivalent embodiments are all included in the scope of the present application defined by the claims. Although embodiments of that present application may be embodied in many different manners, some embodiments of the present application are described in detail herein. In addition, the embodiments of the present application include any possible combination of some or all of the various embodiments described herein, which are also included in the scope of the present application as defined by the claims. All patents, patent applications and other cited information mentioned anywhere in the present application or any of the cited patents, cited patent applications or other cited information are hereby incorporated by reference in their entirety.

The above disclosure is intended to be illustrative rather than exhaustive. For those skilled in the art, this description will suggest many variations and alternatives. All such alternatives and variations are intended to be included within the scope of the claims, wherein the term "including" means "including, but not limited to".

The description of alternative embodiments of the present application is completed herein. Those skilled in the art will recognize other equivalent transformations of the embodiments described herein, which are also included by the claims appended hereto.

## Claims

1. A preparation method of lanthanum carbonate tetrahydrate, comprising the following steps:
(1) reacting lanthanum oxide with acetic acid to obtain lanthanum acetate;
La₂O₃+CH₃COOH → La(CH₃COO)₃
(2) reacting the lanthanum acetate obtained in step (1) with potassium carbonate or potassium bicarbonate or ammonium bicarbonate to obtain lanthanum carbonate octahydrate;
La(CH₃COO)₃ → La₂(CO₃)₃·8H₂O
(3) drying the lanthanum carbonate octahydrate obtained in step (2) to obtain lanthanum carbonate tetrahydrate;
La₂(CO₃)₃·8H₂O → La₂(CO₃)₃·4H₂O

2. The preparation method according to claim 1, wherein the weight ratio of lanthanum oxide to acetic acid in step (1) is 1: 1 to 1: 1.3, preferably 1: 1.1 to 1: 1.2.

3. The preparation method according to claim 1 or 2, wherein the reaction temperature in step (1) is 40 °C to 100 °C, preferably 60 °C to 80 °C; the reaction time is 0.1 h to 2 h, preferably 0.5 h to 1 h.

4. The preparation method according to any one of claims 1 to 3, wherein the weight ratio of lanthanum acetate to potassium carbonate or potassium bicarbonate or ammonium bicarbonate in step (2) is 1: 1.2 to 1: 1.8, preferably 1: 1.4 to 1: 1.5.

5. The preparation method according to any one of claims 1 to 4, wherein the reaction temperature in step (2) is 30 °C to 60 °C, preferably 40 °C to 50 °C; the reaction time is 1 h to 4 h, preferably 2 h to 3 h.

6. The preparation method according to any one of claims 1 to 5, wherein the temperature for the drying in step (3) is 60 °C to 90 °C, preferably 70 °C to 80 °C.

7. Lanthanum carbonate tetrahydrate obtained by the preparation method according to any one of claims 1 to 6.

8. The lanthanum carbonate tetrahydrate according to claim 7, the X-ray powder diffraction (XRPD) pattern thereof is substantially as shown in Fig. 3.

9. The lanthanum carbonate tetrahydrate according to claim 7 or 8, the thermogravimetric analysis graph thereof is substantially as shown in Fig. 4.

10. The lanthanum carbonate tetrahydrate according to any one of claims 7 to 9, wherein the lanthanum carbonate tetrahydrate has a sodium content of less than 0.005 wt%.

11. The lanthanum carbonate tetrahydrate according to any one of claims 7 to 10, the terahertz spectrum thereof has no characteristic spectral peak between 1.0 THz and 3.0 THz and has characteristic spectral peaks between 3.0 THz and 3.9 THz.
